# EUROPÄISCHE PATENTANMELDUNG

(11) **EP 0 533 020 A1**
(43) Veröffentlichungstag der Anmeldung: **24.03.1993**
(21) Anmeldenummer: 92115303.7
(22) Anmeldetag: 08.09.1992
(51) Int. Cl.: G01N 1/20, A01J 5/04, G01N 33/04

(54) **Verfahren zum Erstellen einer für ein Milchvolumen repräsentativen Milchprobe**

(30) Priorität: 16.09.1991 DE 4130732
(71) Anmelder: AHRENS & BODE GmbH & CO. Maschinen- und Apparatebau, D-38364 Schöningen (DE)
(72) Erfinder: Malitz, Horst Dr., Dipl.-Ing., W-3338 Schöningen (DE)
(74) Vertreter: Einsel, Martin, Dipl.-Phys.

(57) **Zusammenfassung**

Ein Verfahren zum Erstellen einer für ein Milchvolumen repräsentativen Milchprobe, in einer insbesondere in einem Milchsammelwagen angeordneten Milchumfüllanlage wird vorgeschlagen. Während des Umfüllens der Milch von einem Anlieferungsbehälter in einen Hauptsammelbehälter werden gleichzeitig oder nacheinander zwei Probeentnahmebehältern (3,5) Milch aus dem Anlieferungsbehälter zugeführt. Einem der Probeentnahmebehälter (3) wird jeweils die Milch aus dem nur dem aktuellen Anlieferungsbehälter zugeführt, während dem zweiten Probeentnahmebehälter (5) summierend von jedem der während der Füllung des Hauptsammelbehälters angesetzten Anlieferungsbehälter eine volumenrepräsentative Probe zugeführt wird.

## Beschreibung

Die Erfindung betrifft ein Verfahren zum Erstellen einer für ein Milchvolumen repräsentativen Milchprobe in einer, insbesondere in einem Milchsammelwagen angeordneten, Milchumfüllanlage, bei dem während des Umfüllens der Milch von einem Anlieferungsbehälter in einen Hauptsammelbehälter gleichzeitig oder nacheinander zwei Probeentnahmebehältern Milch aus dem Anlieferungsbehälter zugeführt wird. Sie betrifft ferner eine Probeentnahmevorrichtung für eine, insbesondere in einem Milchsammelwagen angeordnete, Milchsammeleinrichtung, bei der neben einem Hauptsammelbehälter zwei Probeentnahmebehälter vorgesehen sind.

Die von einzelnen Lieferanten in milcherzeugenden Betrieben gewonnene Milch wird üblicherweise mit Lastkraftwagen, sogenannten Milchsammelwagen, gesammelt und in Molkereien transportiert.

Die Milchsammelwagen sind mit Volumenmessern und Probeentnahmevorrichtungen ausgerüstet, die es ermöglichen, mehr oder weniger exakt, eine repräsentative Probe für die vom jeweiligen milcherzeugenden Betrieb abzuliefernde Milch zu erstellen. Bekannt sind derartige Milchsammelwagen beispielsweise aus der DE 22 45 487 C3 oder der DE 38 23 791 A1.

Eine ganze Reihe von Vorschlägen sind bereits gemacht worden, um die Repräsentativität der Probe eines einzelnen Lieferanten zu verbessern. So wurde beispielsweise in der DE 35 35 179 C1 vorgeschlagen, die Probeentnahme in zwei Teilströmen unterschiedlich großen Probeentnahmebehältern zuzuführen und während des weiteren Verlaufs der Probeentnahme bei dem Lieferanten wieder zusammenzumischen. Mit dieser Maßnahme soll dem Effekt entgegengewirkt werden, daß Milch keine homogene Flüssigkeit ist und bestimmte wertvollere Bestandteile (Sahne) daher nicht gleichmäßig während des Sammelvorgangs und damit auch während des Probeentnahmevorgangs registriert werden können.

Alle bekannten Maßnahmen des Standes der Technik dienen dazu, nach dem Einsammeln der Milch zu einer möglichst gerechten Bewertung der einzelnen milcherzeugenden Betriebe zu kommen.

Molkereien und milchverarbeitende Betriebe werten diese Proben nach der Anlieferung entsprechend aus. Dabei stellt sich gelegentlich heraus, daß für einen bestimmten milchverarbeitenden Betrieb, der beispielsweise aus der Rohmilch Käse oder Yoghurt herstellen möchte, die ihm angelieferte Milch nicht geeignet ist, da sie einen zu hohen Anteil an Antibiotika, Herbiziden oder sonstigen Hemmstoffen enthält. Diese Hemmstoffe, deren Anzahl im allgemeinen bei weitem unterhalb der für Nahrungsmittel bedenklichen Grenzwerte liegen, können das Wachstum von Bakterien behindern, die für die Yoghurtproduktion erforderlich sind. Die gesamte angelieferte Milch ist dann für diesen Betrieb wertlos und kann im günstigsten Falle noch an einen anderen Betrieb weitertransportiert werden, der evtl. andere Kriterien für die von ihm benötigte Milch hat.

Aufgabe der Erfindung ist es demgegenüber, ein Verfahren und eine hierzu geeignete Vorrichtung vorzuschlagen, Molkereien und milchverarbeitende Betriebe werten diese Proben nach der Anlieferung entsprechend aus. Dabei stellt sich gelegentlich heraus, daß für einen bestimmten milchverarbeitenden Betrieb, der beispielsweise aus der Rohmilch Käse oder Yoghurt herstellen möchte, die ihm angelieferte Milch nicht geeignet ist, da sie einen zu hohen Anteil an Antibiotika, Herbiziden oder sonstigen Hemmstoffen enthält. Diese Hemmstoffe, deren Anzahl im allgemeinen bei weitem unterhalb der für Nahrungsmittel bedenklichen Grenzwerte liegen, können das Wachstum von Bakterien behindern, die für die Yoghurtproduktion erforderlich sind. Die gesamte angelieferte Milch ist dann für diesen Betrieb wertlos und kann im günstigsten Falle noch an einen anderen Betrieb weitertransportiert werden, der evtl. andere Kriterien für die von ihm benötigte Milch hat.

Aufgabe der Erfindung ist es demgegenüber, ein Verfahren und eine hierzu geeignete Vorrichtung vorzuschlagen, mit denen die Zeitdauer verkürzt werden kann, die zum Erkennen der Unbrauchbarkeit von Milch für einen bestimmten milchverarbeitenden Betrieb festgestellt wird.

Diese Aufgabe wird beim gattungsgemäßen Verfahren dadurch gelöst, daß einem der Probeentnahmebehälter jeweils die Milch aus nur dem aktuellen Anlieferungsbehälter zugeführt wird, während dem zweiten Probeentnahmebehälter summierend von jedem der während der Füllung des Hauptsammelbehälters angesetzten Anlieferungsbehälter eine volumenrepräsentative Probe zugeführt wird.

Ein hierzu geeignete gattungsgemäße Probeentnahmevorrichtung zeichnet sich dadurch aus, daß einer der Probeentnahmebehälter als Gesamtprobebehälter mit einer Dosiereinrichtung verbunden ist, welche eine der jeweiligen Annahmemenge proportionale Probemenge dem Gesamtprobebehälter zuleitet, wobei die Dosiereinrichtung so einstellbar ist, daß bei vollständiger Füllung des Hauptsammelbehälters auch der Gesamtprobebehälter gefüllt ist.

Bei dem erfindungsgemäßen Verfahren wird zusätzlich zu den aus dem Stand der Technik bekannten Einzelproben eines jeden Lieferanten eine Gemisch- bzw. Gesamtprobe entnommen. Bei jeder Milchannahme und der Abfüllung der Einzelprobe wird eine zusätzliche Probemenge in einem Gesamtprobebehälter abgefüllt. Die Probemenge ist proportional zu der angenommenen Milchmenge des jeweiligen Lieferanten.

Benötigt werden nur verhältnismäßig geringe Milchmengen. Während die Einzelproben jeweils gut verschlossen und erst beim milchverarbeitenden Betrieb untersucht werden müssen, kann mittels des Gesamtprobebehälters bereits während der Fahrt oder zu bestimmten Zeitpunkten zwischen den Annahmestops eine Untersuchung auf gewünschte Kriterien hin unternommen werden. So kann festgestellt werden, wie hoch der Anteil an Hemmstoffen in der Gesamtprobe und damit zu diesem Zeitpunkt auch im Milchsammelwagentank ist. Dieser Anteil kann dazu herangezogen werden, die weitere Fahrtroute des Milchsammelwagens festzulegen, beispielsweise die von ihm zu beliefernde Molkerei bzw. den zu beliefernden milchverarbeitenden Betrieb. Ist beispielsweise der Hemmstoffanteil niedrig, wird ein yoghurtherstellender Betrieb aufgesucht, im gegenteiligen Fall ein Herstellungsbetrieb von pasteurisierter Milch.

Auch wenn die Gesamtprobe noch nicht auf dem Fahrzeug direkt untersucht wird, ist das Vorhandensein einer Gesamtprobe auch dann von Vorteil, wenn die Gesamtprobe erst in der Molkerei untersucht wird. Von Vorteil ist dies darum, weil unmittelbar nach Ankunft des Fahrzeuges die Gesamtprobe zur Verfügung steht und noch vor dem Abtanken der Milch aus dem Tankfahrzeug untersucht werden kann, um über die weitere Verwendung der Milch in der Produktion zu entscheiden.

Wird die Gesamtprobe erst nach der Ankunft des Fahrzeugs aus dem Tank des Fahrzeugs bzw. beim Abtanken des Fahrzeugs entnommen, dann entsteht notwendigerweise ein relativ hoher Zeitverlust, weil im ersten Fall der Inhalt des Milchtanks durch Einsetzen externer Rührwerke sorgfältig durchmischt werden muß, um dann eine Probe aus dem Milchtank gewinnen zu können, oder im zweiten Fall die gesamte Milch erst über ein Probeentnahmesystem beim Abtanken geführt werden muß, um dann eine Probe der Gesamtmenge der Tankmilch zu erhalten.

Von besonderem Vorteil ist es, wenn der Gesamtprobebebehälter mit einer Meßeinrichtung zur Feststellung chemischer und/oder biologischer Eigenschaften der in dem Behälter enthaltenen Milch verbunden ist.

Ist eine derartige Meßeinrichtung vorgesehen, so kann bereits während der Fahrt eine Untersuchung auf bestimmte Kriterien der angesammelten Milch stattfinden. So kann fortlaufend angezeigt werden, ob beispielsweise der Anteil an Hemmstoffen eine bestimmte Grenze überschreitet oder nicht. Ein entsprechendes Signal kann dem Fahrer des Milchsammelwagens gegeben werden, damit er automatisch entsprechend die weitere Route den Gegebenheiten anpaßt.

Bei einem Verfahren ist es von besonderem Vorteil, wenn aus dem Anlieferungsbehälter zunächst ein Vorlaufgefäß mit einer volumenproportionalen Milchprobe gefüllt und anschließend aus dem Vorlaufgefäß die beiden Probeentnahmebehälter mit Milchproben gefüllt werden.

Eine Probeentnahmevorrichtung zur Durchführung dieses Verfahrens zeichnet sich dadurch aus, daß ein Vorlaufgefäß vorgesehen ist, von dem Leitungen zu dem Gesamtprobebehälter bzw. dem Einzelprobebehälter führen.

Vorlaufgefäße, auch als Vorstapelgefäße bezeichnet, sind an sich bekannt. Gerade im vorliegenden Falle erleichtern bzw. verbessern sie die Möglichkeit, die Repräsentativität der gezogenen Milchprobe gerade auch für den Gesamtprobebehälter zu verbessern.

Im folgenden wird anhand der Zeichnung ein Ausführungsbeispiel der Erfindung im einzelnen erläutert.

Es zeigen:
- Fig. 1: eine Schemazeichnung; und
- Fig. 2: eine Schemazeichnung mit zusätzlichen Einzelheiten.

Eine Milchleitung 1 führt die Milch vom Lieferanten (nicht dargestellt) dem Tank (ebenfalls nicht dargestellt) eines Milchsammelwagens zu. Aus dieser Milchleitung 1 wird eine volumenproportionale Milchmenge entnommen und zunächst einem Vorlaufgefäß 2 zugeführt, und zwar über eine Leitung 11. Nach Ende der Milchannahme bei dem jeweiligen Lieferanten wird aus dem Vorlaufgerät 2 in bekannter Weise eine Milchmenge entnommen und in den Einzelprobebehälter 3 abgefüllt, der über ein Ventil 12, eine Leitung 13 und ein weiteres Ventil mit dem Vorlaufgefäß 2 verbunden ist.

Zusätzlich wird im anteiligen Verhältnis des angenommenen Milchvolumens zum Gesamtinhalt des Fahrzeugtanks aus dem Vorlaufgefäß 2 eine Milchmenge entnommen und mit Hilfe einer Dosierpumpe 4 einem Gesamtprobebehälter 5 zugeführt. Ein Ventil 22 und eine Leitung 23 verbinden das Vorlaufgerät 2 mit der Dosierpumpe 4, von der eine Leitung 24 über ein Dreiwegeventil 25 zum Gesamtprobehälter 5 führt.

Die Gesamtprobe (oder Gemischprobe) wird also zusätzlich parallel zu der üblichen Einzelprobenabfüllung angeordnet. Dabei ist es besonders vorteilhaft, daß durch einfache Proportionaleinstellung der angenommenen Milchmenge zum Gesamttankinhalt automatisch auch die in dem Gesamtprobebehälter 5 angesammelten Anteile der einzelnen Milchannahmestellen untereinander korrekt vorhanden sind. Dies gilt auch dann, wenn etwa der Milchsammelwagen nicht ganz gefüllt werden sollte, da das gleiche dann auch für den Gesamtprobebehälter gilt.

Die Dosierpumpe 24 ist vorzugsweise eine impulsgesteuerte Membrandosierpumpe. Die Impulsrate und die Dosiermenge je Hub sind veränderbar und werden so eingestellt, daß bei vollständiger Befüllung des Milchsammelwagentanks der Gesamtprobebehälter 5 unter der vorgegebenen Menge befüllt ist.

Vom Einzelprobebehälter 3 und vom Gesamtprobebehälter 5 führen weitere Leitungen 15 bzw. 26 über Ventile 16 bzw. 27 zu einem Luftabscheider 6. In den Luftabscheider 6 führt (nicht dargestellt) auch die Milchleitung 1. Der Luftabscheider 6 ist wie bei bekannten Milchsammelwagen dem Tank vorgeschaltet. Durch die Verbindungen 15 bzw. 26 kann das Vorlaufgefäß 2 mit der nicht benötigten Milchprobemenge entleert und auch ein Vor- und Nachspülen der Leitungen 13, 23 und 24 erfolgen.

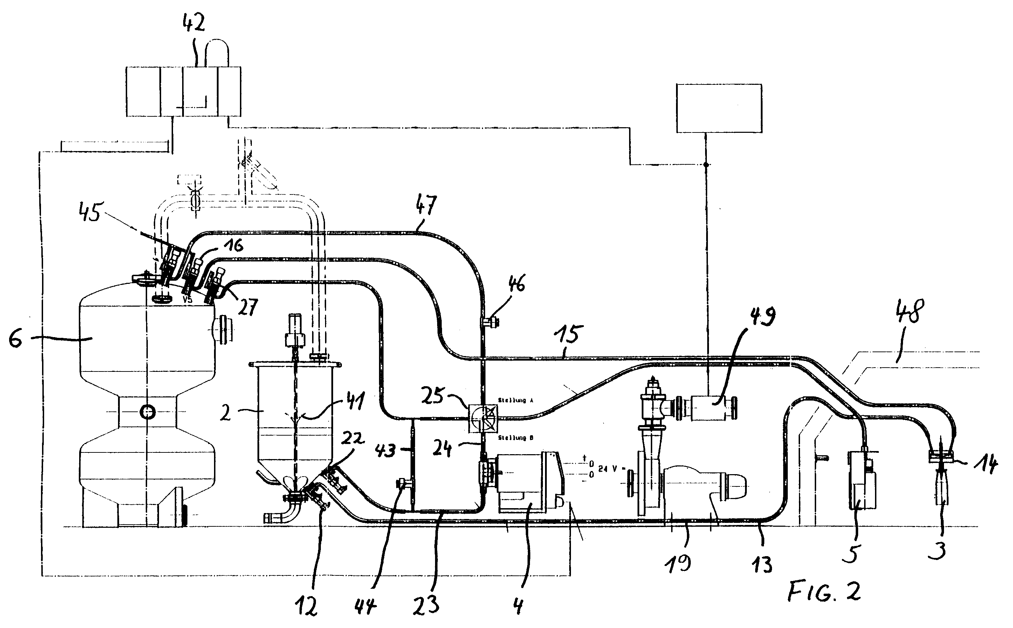

Die Einzelheiten dieses Vorgangs werden im folgenden zusammen mit der Beschreibung der Fig. 2 näher erläutert.

Während der Milchannahme wird Milch über die Entnahmeeinrichtung in das Vorlaufgefäß 2 geleitet, wo die eingeflossene Milchmenge von einem Rührwerk 41 durchmischt wird. Das Vorlaufgefäß faßt größenordnungsmäßig etwa 30 l. Eine Kreiselpumpe 19 fördert die Milch vom Luftabscheider 6 in den Fahrzeugtank. Ein Durchflußzähler 49 ist vorgesehen und seine elektrischen Zählimpulse werden einem Summierspeicher 42 zugeführt.

Nach Beendigung der Milchannahme und beim Auslösen des Abfüllvorgangs des Einzelprobehälters 3 und des Gesamtprobehälters 5 werden die beiden Schlauchleitungen 13 und 23, die zum einen zum Ventil 14 und zum anderen zur Membrandosierpumpe 4 führen, zunächst durchgespült.

Dazu werden die Ventile 12 bzw. 22 geöffnet und die Ventile 16 und 27 zum Luftabscheider 6 umgeschaltet. Mit Hilfe des Vakuums wird Milch durch die Schlauchleitungen 13 und 23 gesaugt. Während des Vorspülens sind in einer Ausgleichszweigleitung 43 parallel zur Dosierpumpe 4 und zum Ventil 25 ein Ventil 44 geschlossen, das Vierwegedrehventil 25 in Stellung "A" geschaltet und die Dosierpumpe 4 eingeschaltet. Dadurch wird die angesaugte Milchmenge gezwungen, durch die Dosierpumpe 4 zu fließen. Die Membrankammer der Dosierpumpe 4 wird entlüftet und evtl. Milchreste der vorherigen Probenahme werden aus den Schläuchen 13, 23 und aus der Membrankammer gespült. Kurz vor Ende des Spülvorganges wird das Ventil 44 geöffnet, so daß auch die Ausgleichszweigleitung 43 entlüftet und gespült wird. Zum Ende des Spülvorganges schließen die Ventile 12 und 22. Die Ventile 16 und 27 werden in die Stellung "belüften" umgeschaltet.

Der Einzelprobebehälter 3, eine Probeflasche, wird über das Ventil 12, die Leitung 13 und das Ventil 14 abgefüllt.

Die Abfüllung der Gemisch- oder Gesamtprobe erfolgt zweckmäßig nach Beendigung der Abfüllung in den Einzelprobebehälter 3.

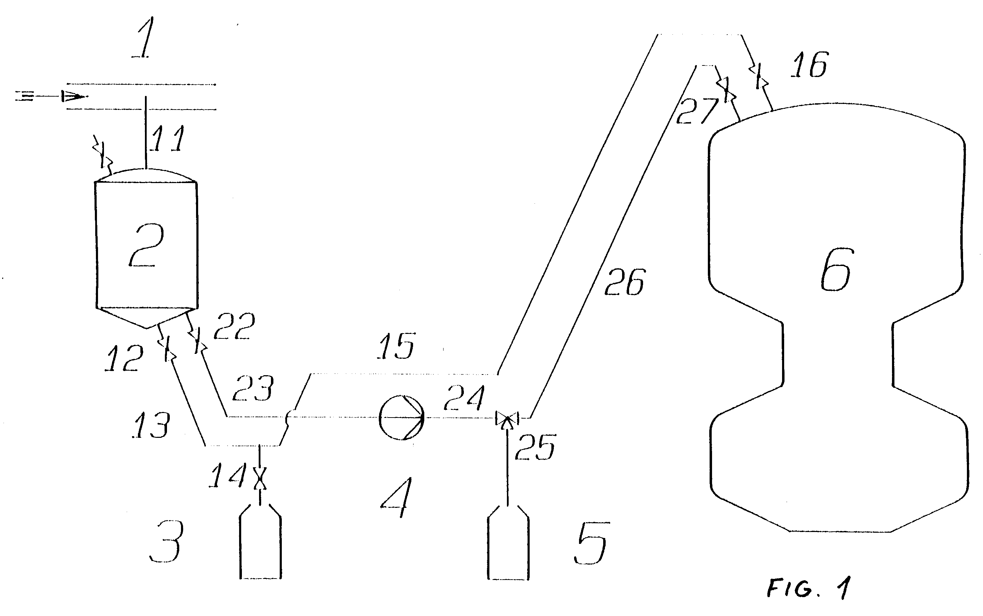

Die Schlauchleitung 23 von dem Ventil 22 zu der Dosierpumpe 4 und weiter zum Ventil 27 ist mit Milch aus dem Vorlaufgefäß 2 vorgespült und befüllt worden. Damit die Dosierpumpe 4 die Probemengen zu dem Gesamtprobebehälter 5 hin abpumpen kann, wird jetzt das Vierwegedrehventil 25 (in Fig. 1 vereinfacht als Dreiwegeventil dargestellt) in die Stellung "B" umgeschaltet. Das Ventil 42 bleibt weiter geöffnet, so kann die Dosierpumpe 4 aus den Schlauchleitungen 23 und 43 ansaugen.

Dazu wird die Dosierpumpe 4 eingeschaltet, wobei sie von dem Summierspeicher 42 eine vorbestimmte Anzahl von Impulsen erhält und entsprechend diesen Impulsen die Pumphübe ausführt. Die Anzahl der Impulse ist dabei proportional zu dem Inhalt des Summierspeichers 42 und damit proportional zur einzelnen Milchmenge.

Nachdem die Dosierpumpe 4 die vorgegebenen Hübe abgearbeitet hat, wird das Vierwegedrehventil 25 wieder in die Stellung "A" zurückgeschaltet. Danach wird mit Hilfe von Druckluft die Schlauchleitung 24, in der sich noch die dosierte Probemenge befindet, durch die Abfüllnadel in den Gesamtprobebehälter 5 gedrückt. Die Druckluft wird aus der ölfreien Druckluftversorgung über das Ventil 45 herangeführt. Dazu öffnet das Ventil 46.

Während noch die Probemenge in den Gesamtprobebehälter 5 gedrückt wird, beginnt gleichzeitig das Leersaugen der Dosierpumpe 4 mit Hilfe des Vakuums im Luftabscheider 6. Dazu wird das Ventil 22 geöffnet und das Ventil 27 zum Luftabscheider hin umgeschaltet. Die Dosierpumpe 4 wird erneut eingeschaltet und das Ventil 42 geschlossen, so daß zunächst die Membrankammer entleert wird. Vor Ende des Absaugvorganges wird das Ventil 42 wieder geöffnet, so daß auch die Leitung 43, die Ausgleichs-Schlauchleitung, geleert wird.

Zur Reinigung öffnet das Ventil 12 und das Ventil 16 schaltet um zum Luftabscheider 6, damit die dazwischenliegende Leitung 13 von der Reinigungsflüssigkeit durchströmt werden kann. Das Ventil 14 wird dabei geöffnet, so daß auch die Nadel mit gereinigt wird.

Die Reinigung der Dosierpumpe 4 erfolgt in der Weise, daß das Ventil 12 öffnet und das Ventil 45 in die Stellung zum Luftabscheider 6 umschaltet, damit die auch hier dazwischenliegende Leitung 23 von der Reinigungsflüssigkeit durchströmt wird. Hierbei wird auch die Dosierpumpe 4 in Betrieb gesetzt, so daß auch die Membrankammer durchspült wird.

Außerdem werden das Ventil 45 zum Luftabscheider 6 umgeschaltet und das Ventil 46 geöffnet, so daß die Reinigungsflüssigkeit auch durch die dazwischenliegende Leitung 47 fließen kann und schließlich über die Leitung 26 zu der Abfüllnadel des Gesamtprobebehälters 5 gelangt. Das Vierwegedrehventil 25 bleibt während der Reinigung in Stellung "A".

Auf beide Abfüllnadeln werden während der Reinigung Schläuche aufgesteckt, um die evtl. austretenden Reinigungsflüssigkeiten abzuführen.

In der Fig. 2 sind die Ventile 12 und 22 versetzt dargestellt. In der Realität wird es bevorzugt, wenn diese niveaugleich angeordnet sind. Der Einzelbehälter 3 und der Gesamtprobebehälter 5 sind gemeinsam in einer isolierten Probekabine 48 untergebracht. Die Dosierpumpe 4 wird durch mengenabhängige Impulse angesteuert, die von dem Durchflußzähler 49 abgegeben und im Summenspeicher 42 gesammelt werden.

## Patentansprüche

1. Verfahren zum Erstellen einer für ein Milchvolumen repräsentativen Milchprobe in einer, insbesondere in einem Milchsammelwagen angeordneten, Milchumfüllanlage, bei dem während des Umfüllens der Milch von einem Anlieferungsbehälter in einen Hauptsammelbehälter (bei 6) gleichzeitig oder nacheinander zwei Probeentnahmebehältern (3,5) Milch aus dem Anlieferungsbehälter zugeführt wird, **dadurch gekennzeichnet**, daß einem der Probeentnahmebehälter (3) jeweils die Milch aus nur dem aktuellen Anlieferungsbehälter zugeführt wird, während dem zweiten Probeentnahmebehälter (5) summierend von jedem der während der Füllung des Hauptsammelbehälters (bei 6) angesetzten Anlieferungsbehälter eine volumenrepräsentative Probe zugeführt wird.

2. Verfahren nach Anspruch 1, **dadurch gekennzeichnet**, daß aus dem Anlieferungsbehälter zunächst ein Vorlaufgefäß (2) mit einer volumenproportionalen Milchprobe gefüllt und anschließend aus dem Vorlaufgefäß (2) die beiden Probeentnahmebehälter (3,5) mit Milchproben gefüllt werden.

3. Probeentnahmevorrichtung für eine, insbesondere in einem Milchsammelwagen angeordnete, Milchsammeleinrichtung, bei der neben einem Hauptsammelbehälter (bei 6) zwei Probeentnahmebehälter (3,5) vorgesehen sind, **dadurch gekennzeichnet**, daß einer der Probeentnahmebehälter (5) als Gesamtprobebehälter mit einer Dosiereinrichtung verbunden ist, welche eine der jeweiligen Annahmemenge proportionale Probemenge dem Gesamtprobebehälter (5) zuleitet, wobei die Dosiereinrichtung (6) so einstellbar ist, daß bei vollständiger Füllung des Hauptsammelbehälters (bei 6) auch der Gesamtprobebehälter (5) gefüllt ist.

4. Probeentnahmevorrichtung nach Anspruch 3, **dadurch gekennzeichnet**, daß der Gesamtprobebehälter (5) mit einer Meßeinrichtung zur Feststellung chemischer und/oder biologischer Eigenschaften der in dem Behälter (5) enthaltenen Milch verbunden ist.

5. Probeentnahmevorrichtung nach Anspruch 3 oder 4, **dadurch gekennzeichnet**, daß ein Vorlaufgefäß (2) vorgesehen ist, von dem Leitungen (13,15; 23,26) zu dem Gesamtprobebehälter (5) bzw. dem Einzelprobebehälter (3) führen.
